# EUROPEAN PATENT SPECIFICATION

(11) **EP 1 226 432 B1**
(45) Date of publication and mention of the grant of the patent: **07.06.2006**
(21) Application number: 99952548.8
(22) Date of filing: 11.10.1999
(51) Int. Cl.: G01N 33/497, G01N 25/48

(54) **DIFFERENTIAL GAS MEASUREMENT, IN PARTICULAR FOR BREATHING-GAS ANALYSIS**
DIFFERENZIELLE GASMESSUNG, INSBESONDERE UM ATEMGAS ZU ANALYSIEREN
MESURE DIFFERENTIELLE DE GAZ, EN PARTICULIER, POUR ANALYSER UN GAZ RESPIRATOIRE

(43) Date of publication of application: 31.07.2002
(73) Proprietor: Koninklijke Philips Electronics N.V., 5621 BA Eindhoven (NL)
(72) Inventor: WUNDERLING, Martin, D-71083 Herrenberg (DE); FISCHER, Bernhard, D-71229 Leonberg (DE)
(74) Representative: Volmer, Georg
(86) International application number: PCT/EP1999/007602
(87) International publication number: WO 2001/027615

(56) References cited:
- EP-A- 0 459 284
- WO-A-84/01704
- WO-A-99/43388
- US-A- 5 094 235
- US-A- 5 177 464
- US-A- 5 619 986
- US-A- 5 789 660
- "Encyclopedia of PHYSICAL SCIENCE AND TECHNOLOGY" 1987, ACADEMIC PRESS INC , ORLANDO, FLORIDA

## Description

### BACKGROUND OF THE INVENTION

The present invention relates to monitoring of a gas flow such as breathing gases.

Monitoring of breathing gases of patients under general anesthesia has become standard clinical practice. The gas monitors used for this purpose are calibrated for a limited number of gases, e.g. oxygen, carbon dioxide, nitrous oxide, and a number of anesthetic agents such as halothane, isoflurane, enflurane, sevoflurane and desflurane.

Other gaseous components in the breathing gas mixture are typically not identified and their presence can in some cases lead to erroneous readings of the monitored gases due to cross interference effects. In other cases, the non-identified gaseous components can become dangerous for the patient especially if the concentration exceeds certain limits. Examples for both cases are e.g. methane originating from the patient's stomach which can lead to wrong readings of anesthetic agent concentrations, and carbon monoxide created inside the anesthesia machine which has a large affinity to the hemoglobin in blood and is therefore highly toxic, as reported by Peter B. Berry et al. in *Severe Carbon Monoxide Poisoning during Desflurane Anesthesia,* Anesthesiology V 90, No. 2, Feb 1999, p. 613. The extremely poisonous gas nitric oxide is administered to patients typically at concentrations below about 100 ppm for therapeutic purposes; it is avidly absorbed within the lungs (cf. GG Lavery, *Delivery Systems & Clinical Indications for Nitric Oxide,* in: Association for Low Flow Anesthesia Meeting Abstracts, Belfast 1997).

US 5,619,989 show a reinhalation system for an anesthetic apparatus including exhalation and inhalation branches, which are directly connected to each other. The inhalation branch comprises a dosing unit capable of supplying a component to said inhalation branch. A control unit controls the dosing unit. A first concentration measuring device measuring the concentration of the component and a flow meter measuring a flow rate of a fluid are provided. The dosing unit suppliers at least the component to the inhalation branch at a dosing point located downstream of the first concentration meter. The control unit controls the supply of the component as a function of the concentration of the component measured by the concentration meter and the flow rate of the fluid measured by the flow meter.

By the publication of " Encyclopedia of Physical Science and Technology" Vol. 12, 1987, Academic Press INC; page 49-50 the general reactor design equation and ideal batch reactor equation are known.

US 5,177,464 disclose an on-board monitoring system for an automotive emission catalyst system. The system comprises a test chamber remote from the exhaust stream. The test chamber is connected to the gas stream by a channel upstream and by a further channel downstream to the catalytic converter. The system comprises valve means to alternatively measure the concentration of hydrocarbon of a gas probe up- and downstream to the catalytic converter. The sensed signals are compared and if the difference fall below a predetermined difference value the catalyst is indicated as faulty.

### SUMMARY OF THE INVENTION

It is therefore an object of the present invention to provide an improved gas measurement which can be preferably used for early identification of small quantities of gaseous components, thus enabling e.g. to issue a timely warning such that the clinical personnel can react before any harm is done to the patient.

The object is solved by the independent claims. Preferred embodiments are shown by the dependent claims.

The invention allows determining whether a monitored object increases or decreases the concentration of a gas component in a gas flow, so that conclusions can be drawn back about the source and/or nature of the gas component. In particular in medical applications, this allows distinguishing e.g. for one and the same determined concentration of a gas component whether this concentration might cause harm to the patient (since the patient will absorb and accumulate even small quantities 'supplied' to the patient) or is harmless (since it 'originates' from the patient).

The invention makes use of the observation in medical applications that some gas species accidentally or intentionally fed to the patient are absorbed or accumulated within the patient. Other species may originate from within the patient and can be eliminated e.g. by an anesthesia ventilation equipment. In either case, there is a difference in concentration of this species between the inhaled and exhaled gas stream. Determining the concentration on one side of the gas flow from or to the patient only, however, can lead to erroneous interpretations of the measurement. In case that the patient emits the gas species (e.g. a smoker exhaling a certain amount of CO), the measurement can be misinterpreted in that there must be source of the species other than the patient (e.g. an anesthetic agent degradation). Since some gas species can be rather toxic, such a misinterpretation can lead to an immediate abort of the respiration process. In case that the patient absorbs the gas species (e.g. CO originating from an anesthetic agent degradation), this might not be detected at all or simply not be recognized due to only small concentrations of the species.

According to the invention, a differential (also referred to as relative) gas measurement of the inhaled and exhaled gas stream is applied. The relative concentration of a gas component is detected by determining the relative difference in concentration of the gas component between the inhaled and exhaled gas stream. If the concentration of the gas component in the inhaled gas stream is smaller than in the exhaled gas stream, the patient himself must be the source of the gas component. On the other hand, if the concentration of the gas component in the inhaled gas stream is greater than in the exhaled gas stream, the patient himself must at least partly absorb the gas component.

In general, the invention provides qualitative analyses of the gas stream by determining whether the monitored objects increases or decreases the concentration of the gas component in the gas flow. In other words, the invention allows qualitatively determining whether between two measuring points (e.g. one for the inhaled and one for the exhaled gas stream), a particular gas component:
- remains unchanged in concentration, or
- is expelled, emitted, blown out, taken out, or otherwise added into the gas stream, or
- is absorbed, depleted, exhausted, taken in or up, or otherwise removed from the gas stream.

The invention thus allows determining an emission or absorption of gas components in a gas flow by the object. Threshold values for emission or absorption can be set and alarms or other predefined actions can be initiated when one or more of the threshold values are exceeded.

Applying those principles e.g. on the detection of CO, a CO concentration in the inhaled gas stream higher than in the exhaled gas stream indicates absorption and retention of CO inside the patient. An onset of CO production e.g. by degradation of the anesthetic agent (cf. the co-pending International Application WO 01/15762 A1 [PCT/EP99/06348] by the same applicant) can thus be detected.

In most measuring setups, the lower detection limit for a given gas (e.g. in the inhale or exhale stream) is dependent not only on signal noise but also on baseline drifts. Baseline drifts can usually be corrected to a certain extend by occasional "zero calibrations", especially during a warm-up phase of the equipment. The differential detection according to the invention, however, is of higher sensitivity against small changes even at very low concentrations, because it is independent of small background concentrations, e.g. in case of CO background concentrations stemming from previous tobacco smoking of the patient. The relative detection is also independent of baseline drifts of the measurement equipment, since those baseline drift effects disappear in the differential analysis, e.g. by subtraction.

Significantly increasing sensitivity of the differential detection according to the invention can be achieved by integrating over several inhalation phases and, accordingly, over substantially the same number of exhalation phases. By a subsequent subtraction of the integrated inhaled from the integrated exhaled gas signals, even small concentrations of such species can be determined with high sensitivity, thus also increasing the signal to noise ratio.

When measuring at the same location in the gas stream, differentiation between inhalation and exhalation and, thus, determining time windows for the inhale and exhale phases can be obtained by applying techniques as well known in the art. Preferably, CO₂ wave patterns available from a gas monitor can be applied. In that case, presence of CO₂ will be indicative of exhalation, and, accordingly, absence of CO₂ will be indicative of inhalation. Further more, a direct measurement of flow direction could be used in a gas monitor equipped with a flow measurement system to determine the time windows of inhale and exhale phases.

In a preferred embodiment, the signals of the gases of interest are selectively measured within suitable time windows within the inhale phase and exhale phase, respectively. The signals are integrated and averaged over a multitude of such time windows separately for the inhale and exhale phases in order to improve the signal-to-noise ratio. By a subsequent subtraction of the inhaled from the exhaled gas signals, even small concentrations of such species can be determined with high sensitivity. The gas monitor has to be calibrated for the gas species in question for using this method.

The invention preferably applies Raman scattering for gas analyzing purposes. Details about Raman scattering are readily applicable from the aforementioned co-pending International Application WO01/15762 A1 [PCT/EP99/06348].

Gas analyzers employing Raman spectroscopy can be calibrated to various Raman-active gases. The spectral "fingerprint" of Raman-active gases can be used to identify constituents of even very complex gas mixtures, and the relative intensity of the spectral contributions by each member gas is used to quantify the gases. Such a gas analyzer can be calibrated e.g. to methane, carbon monoxide, alcohols, and many other species of interest in addition to the usual respiratory and anesthetic gases. In particular, nitrogen can also be detected by a Raman gas monitor.

Preferably, a Raman gas analyzer is employed using a laser source in the visible spectral region to excite the Raman spectrum, and which is equipped with a spectrometer to measure the Raman lines preferably in a spectral range of about 200 nm from the excitation wavelength. This gas analyzer can be calibrated for any "Raman-active" gas by exposing the Raman measurement cell to a pure sample (or diluted mixture) containing this gas and recording the respective Raman spectrum as a calibration spectrum.

The following few examples may serve as illustrations of the advantages gained by the use of the invention:
- A gas monitor as described above having the early warning capability against unwanted exposure of a patient to gases like carbon monoxide, CO, has the clear advantage that accidental CO poisoning can be reliably avoided.
- The early detection of nitrogen in a breathing mixture which is not supposed to contain nitrogen is a clear indicator of either a leak in the breathing circuit or of air flux into the blood stream through a large wound open to the ambient air. Such air influx is known to cause air emboli that can be lethal if they reach the small blood vessels in the lung. The early detection of very small quantities of exhaled nitrogen can indicate the danger of an air embolism in the latter case. Nitrogen found in the inhaled gas would be indicative of a leak in the breathing circuit.
- Nitric oxide, NO, needs to be monitored very closely due to its toxicity both for patients as well as for clinical personnel administering this gas. A gas monitor of the described type would facilitate the supervision of the NO absorption rate into the patient.

Alternatively to the employment of Raman scattering, infrared absorption spectroscopy can also be applied for the purpose of the invention e.g. for the detection of unwanted gas species. However, the larger widths and overlaps in IR absorption bands of the species of interest render the identification task to be more complex than with Raman spectroscopy. Also, not all gases of interest are detectable by IR methods, e.g. nitrogen and oxygen cannot be detected.

The differential gas measurement can be applied to any measurement in a gas flow wherein the gas sampling is provided either
- at the same sampling location (sampling site) but for different directions of the gas flow, whereby the object to be monitored is within the gas flow, or
- at different sampling locations (sampling sites) with the object to be monitored between the sampling locations and with the same direction of the gas flow.

Dependent on the characteristics or nature of the object, the object can either:
- leave the concentration of a specific gas component unchanged in the gas stream, or
- expel, emit, blow out, take out, or otherwise add the specific gas component into the gas stream, thus increasing the concentration of the specific gas component in the gas stream, or
- absorb, deplete, exhaust, take in or up, or otherwise remove the specific gas component from the gas stream, thus decreasing the concentration of the specific gas component in the gas stream.

Since the invention not only determines the concentration of a specific gas component in the gas stream but also whether the object increases or decreases the specific gas concentration, different threshold values can be used dependent on the characteristics of the object or, in other words, dependent whether the gas concentration has been increased or decreased by the object. This allows that for the same concentration of a specific gas component determined in the gas stream, a different threshold value can be defined dependent whether the determined gas concentrations has been increased or decreased by the object. This case-sensitive thresholding allows adapting the threshold values to the 'origin' of the gas component. Alarms or other predefined actions can be initiated when one or more of the threshold values are exceeded.

Moreover, threshold values for absolute gas concentrations in the gas stream can be combined with determined values and/or threshold values for differential gas concentrations, and vice versa. This allows e.g. that the threshold values for differential gas concentrations can be defined dependent on the determined absolute gas concentration.

It is clear that the invention can be partly or entirely embodied by one or more suitable software programs, which can be stored on or otherwise provided by any kind of data carrier and which might be executed in or by any suitable data processing unit.

### BRIEF DESCRIPTION OF THE DRAWINGS

Other objects and many of the attendant advantages of the present invention will be readily appreciated and become better understood by reference to the following detailed description when considering in connection with the accompanied drawings. Features that are substantially or functionally equal or similar will be referred to with the same reference sign(s).
- Fig. 1: depicts the schematic view of a gas monitor 10 according to the invention, and
- Fig. 2: shows an example of a measurement of a composition of a gas mixture with a number of gas constituents.

### DETAILED DESCRIPTION OF THE INVENTION

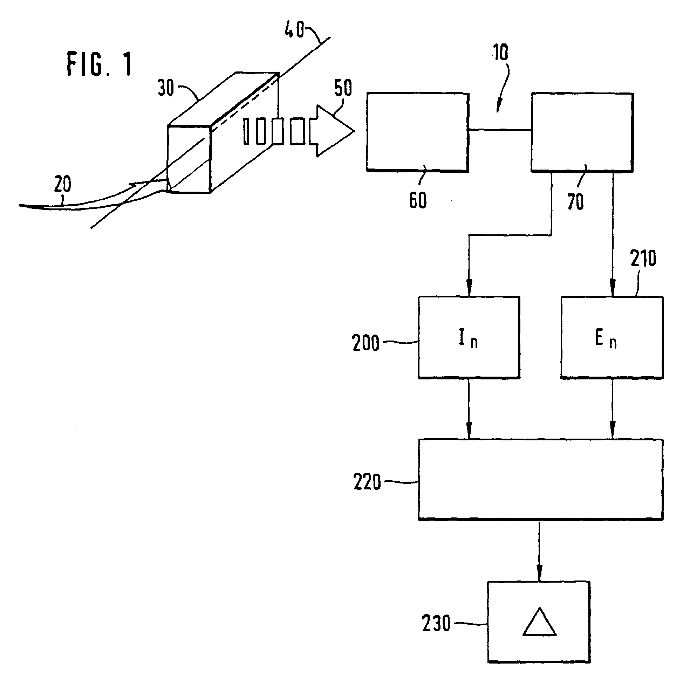

Fig. 1 depicts the schematic view of a gas monitor 10 according to the invention. A gas flow 20 with a gas mixture, such as a respiration gas, is directed through a sample cell 30. An incident light beam 40, e.g. from a laser source, is scattered in the sample cell 30 and a scattering light 50 is received by a spectrograph 60. The spectrograph 60 is further coupled to a processing unit 70 for determining the composition of the gas mixture in the gas flow 20.

The processing unit 70 is preferably further connected (not shown) to the source of the light beam 40 for receiving information about the light beam 40, such as the intensity. The processing unit 70 is preferably further coupled to a (not shown) pressure determining means and a temperature sensor within the sample cell 30 for receiving information about the pressure and temperature therein.

In a first step, the spectrograph 60 of the gas monitor 10 measures the Raman spectrum of the gas mixture. In a second step, the processing unit 70 then determines the quantitative amount of one or more gas components in the gas mixture of the gas flow 20 by comparing the measured Raman spectrum with stored reference spectra for the gas components. Each reference spectrum generally represents the Raman spectrum for the pure gas component, determined under known conditions, e.g. a known condition of pressure and/or temperature within the sample cell 30 and of the intensity of the incident light beam 40. Accordingly, reference spectra can be applied already representing a defined gas mixture. The proportion of the measured spectrum to each reference spectrum provides a direct measure of the proportion of the individual gas component (represented by the reference spectrum) in the gas mixture.

The assignment of the peak(s) in the measured spectrum to the individual gas component(s) can be done as known in the art, e.g. by comparing the wavelength(s) of the peak(s) with the wavelength(s) of the reference spectrum/spectra of the individual gas component(s).

The comparison of the measured Raman spectra with the reference Raman spectra is preferably accomplished by determining the ratio of the amplitudes (intensities) for each wavelength channel of the spectrograph. However, other comparison methods e.g. by means of the peak area or the like can be applied accordingly.

In case that a certain individual gas component reveals more than one Raman line, all lines are usually attenuated substantially evenly, so that, for the purpose of the invention, it is normally sufficient to evaluate only one Raman line for each gas component for determining the proportion of the individual gas component in the gas mixture.

The reference spectra comprising the wavelength positions and intensities are preferably determined by previous measurements and can be stored e.g. in a calibration matrix.

In case that the actual measuring conditions deviate from the measuring conditions of the reference spectra, the measured spectra may have to be corrected, e.g. for the effects of pressure, temperature, and light intensity changes, using well-known algorithms.

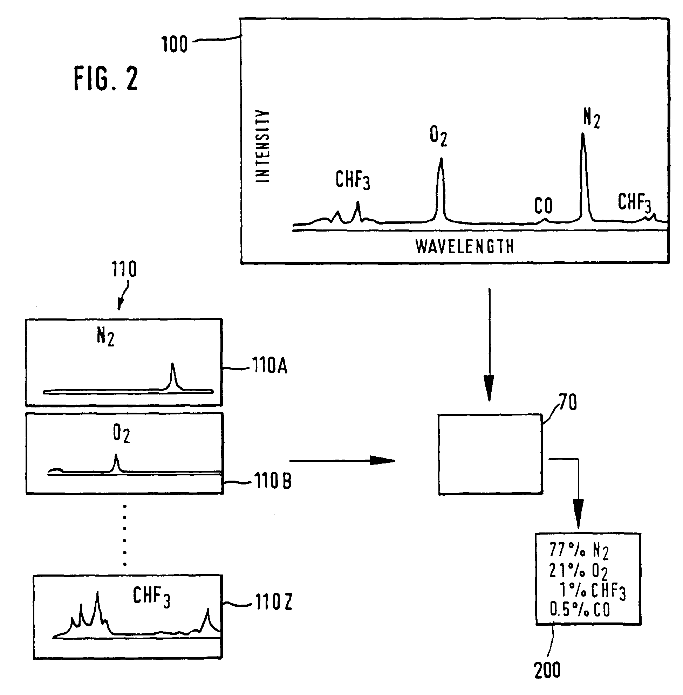

Fig. 2 shows in an example the principles for determining the composition of a gas mixture with a number of gas constituents. The spectrograph 60 measures a Raman spectrum 100 of the gas mixture. The wavelength position and intensities of a plurality of Raman lines are stored in a calibration matrix 110 with a plurality of individual reference spectra 110A...110Z for several gas constituents.

For determining proportions of the individual components in the gas mixture, the measured spectrum 100 of the gas mixture is compared with the respective reference spectra 110A, 1108 of the calibration matrix 110. Thus, the proportions of the peak levels from the reference spectra 110A, 110B, and 110Z to the measured spectrum 100 provides a direct measure for the proportions of the individual components in the gas mixture.

The example of the measured spectrum 100 as depicted in Fig. 2 shall represent the measured spectrum of an inhalation gas sample determined within an inhalation cycle Iₙ. The wavelengths and characteristics of the measured peaks in Fig. 2 refer to N₂, O₂, CHF₃, and CO. In this example, the peak N₂ shall represents 77% of the reference peak for N₂ in the reference spectrum 110A, the peak O₂ represents 21 % of the reference peak for O₂ in the reference spectrum 110B, and both CHF₃ peaks (to the very left and right in the spectrum 100) represents 1 % of the reference peak for CHF₃ in the reference spectrum 110Z. The peak CO represents about 0.5% of the reference peak for CO (not shown in 110). Accordingly, the gas composition of the measured inhalation gas sample with the spectrum 100 is: 77% of N₂, 21% of O₂, 1% of CHF₃, and about 0.5% of CO. The processing unit 70 (cf. Figs. 1 and 2) provides the determined gas composition of the inhaled gas sample as indicated by reference number 200.

The differential detection according to this invention determines the gas constituents of the inhaled and exhaled breathing gases, separately. Determining the proportion of CO₂ and/or O₂ allows differentiating between inhalation and exhalation phases, whereby the presence of CO₂ and/or the reduction of O₂ is indicative of exhalation and, accordingly, absence of CO₂ and/or the presence of O₂ at the unreduced level is indicative of inhalation. More precisely, the time phase of increasing CO₂ concentration and/or decreasing O₂ concentration indicates exhalation, while the time phase with decreasing CO₂ concentration and/or increasing O₂ concentration indicates inhalation. Thus, the CO₂ wave pattern contains the information about the flow direction. A direct flow direction measurement might also be applied. It is clear that the sampling time phases have to be suitably selected in particular dependent on the flow conditions and thus of the intermixing between inhale and exhale gases.

In the example of Fig. 1, the gas flow 20 shall represent the sample flow of gas taken out of the patient breathing tube which contains the inhale or the exhale gas, or both, however at different points in time. In the example of Fig. 1, the inhale and the exhale gases are directed (e.g. by means of corresponding valves) from the same side to the sample cell 30, both with the same direction of flow. This type of side stream sampling is widely used in medical gas monitoring. However, the inhale and exhale gases might also be provided from opposite sides to the sample cell 30 (which has no influence on the Raman measurement). Furthermore, there might be provided one sample cell 30 in the inhale gas stream and one other in the exhale gas stream.

A next gas sample will then be taken for an exhalation cycle Eₙ succeeding the inhalation cycle Iₙ, and a corresponding spectrum (not shown in the Figures) will be determined for that exhale gas sample. In this example, the exhaled gas mixture shall consist e.g. of 77% N2, 16% 02, 5% CO2, 1% of CHF3 and 0.1% CO. Accordingly, the processing unit 70 (cf. Fig. 1) will provide the determined gas composition of the exhaled gas sample as indicated by reference number 210 (cf. Fig. 1).

The values of the gas composition 200 of the inhaled gas for the inhalation cycle Iₙ and the values of the gas composition 210 of the exhaled gas for the corresponding (succeeding) exhalation cycle Eₙ are then provided to a subtraction unit 220. The subtraction unit 220 subtracts the values of the gas composition 200 from the values of the gas composition 210 (or vice versa), thus leading to a differential result 230 representing the change in gas composition between successive inhalation and exhalation cycles.

The differential result 230 might be determined only between corresponding pairs of gas samples, e.g. between the exhale gas sample of Eₙ and the inhale gas sample of Iₙ and next between the exhale gas sample of Eₙ₊₁ and the inhale gas sample of Iₙ₊₁. Alternatively, or in addition thereto, the differential result 230 can also be determined between each successive gas samples, e.g. between the inhale gas sample of Iₙ and the exhale gas sample of Eₙ, next between the exhale gas sample of Eₙ and the inhale gas sample of Iₙ₊₁, then between the inhale gas sample of Iₙ₊₁ and the exhale gas sample of Eₙ₊₁, and so on.

In the above example, subtraction of the values of the gas composition 200 from the values of the gas composition 210 leads - besides the expected difference in O₂ and CO₂ - to a significant difference of 0.4% CO between the inhale and the exhale gas stream, or, in other words, to an amount of 0.4% CO flowing into the patient. In a closed respiration system, this indicates that there must be a source of CO somewhere within the respiration system, e.g. originating from a degradation of an anesthetic agent.

In a preferred embodiment, the sensitivity of the differential gas detection is increased by integrating over several inhalation and exhalation phases. This can be done by either integrating the values of the inhale gas composition 200 and the values of the exhale gas composition 210 for a plurality of (successive) inhale or exhale cycles, respectively, or integrating a plurality of (successive) differential results 230. By a subsequent subtraction of the integrated inhaled from the integrated exhaled gas signals, even small concentrations of such species can be determined with high sensitivity.

In another example, the background signal may have shifted and contributes to the value of the CO signal, both for inhaled and exhaled gases, with +0.4% such that the value of CO inhaled is 0.6% and exhaled is 0.4%. The differential method then still indicates a net flow of 0.2% CO into the patient regardless of whether the exhaled 0.4% is real or represents a measurement artifact.

Threshold values for certain gas types can be set and alarms or other consequences can be initiated when exceeding the set threshold values. Threshold values can be defined for the absolute value of the differential result 230. In addition thereto, different threshold values can be defined dependent on the sign of the differential result 230, or in other words, dependent whether the differential result 230 is positive or negative. In the above example, wherein an amount of +0.4% CO absorbed by the patient has been determined, a first threshold value of +0.3% CO (for absorbing CO) and a second threshold value of -0.5% CO (for emitting CO) can be applied, so that in this case an alarm would be triggered. The threshold values for the differential result 230 can also be defined dependent on the 'absolute' concentration (e.g. in the inhale or exhale gas stream), so that threshold values for the differential result 230 might e.g. be decreased with increasing determined 'absolute' concentrations.

When e.g. the determined quantitative amount of one or more of the anesthetic agent degradation products in the gas mixture exceeds given threshold values for each of the degradation products, an alarm will preferably be given. The determination of reasonable threshold values for the detection of the degradation products is of course dependent on the sensitivity of the specific embodiment of the measurement system. Since one dangerous aspect of CO poisoning is the dose (the dose being the concentration multiplied by the exposure time) deposited in the blood hemoglobin, the optimization of the threshold values should preferably take into account both the detection limits for the degradation products as well as the system's integration time associated with those detection limits. On one hand, it is desirable to have threshold values as low as possible in order to generate a warning as early as possible, but, on the other hand, false-positive alarms triggered in an overly sensitive system are to be avoided, too. In a preferred embodiment, one threshold value for the absolute concentrations of CO of 0.5% may be used and another threshold value of e.g. 0.2% CO for the difference between inhaled and exhaled. The alarm triggered by exceeding the threshold value on the difference is less likely to be false-positive, yet the system is even more sensitive to small real concentrations of CO. A false alarm resulting from the erroneously positive identification of the alarm-triggering event is typical for an overly sensitive system; false-positive alarms are annoying in the first place, but not dangerous. The contrary is false-negative results, where the erroneously determined absence of a dangerous situation does not trigger an alarm and therefore creates a wrong sense of security.

## Claims

1. A method for monitoring a gas flow (20) passing to and from a patient, the method comprising the steps of:
(a) determining a first concentration (200) of a gas component at a first position within the gas flow,
(b) determining a second concentration (210) of the gas component at a second position within the gas flow, wherein
a patient is situated between said first and second position, the first concentration represents an inhale gas concentration of said gas component,
the second concentration represents an exhale gas concentration of said gas component, and
(c) determining (220) a differential concentration (230) of the gas component from the first and second concentrations, whereby the differential concentration indicates whether the concentration of the gas component is increased, decreased or remained substantially unchanged between the first and second positions within the gas flow.

2. The method of claim 1, wherein the first and second positions are provided:
at the same location but with different directions of the gas flow, or
at different locations but in the same direction of the gas flow.

3. The method of claim 1 or 2, wherein:
the first concentration is an inlet concentration before passing an object situated within the gas flow between the first and second positions,
the second concentration is an outlet concentration after passing the object, and
the method further comprises a step of:
(d) determining the characteristic of the object by means of the differential concentration of the gas component, preferably in a way that:
the object represents a source of the gas component in case that the concentration of the gas component is increased, or
the object at least partly removes the gas component from the gas stream in case that the concentration of the gas component is decreased.

4. The method according to any one of the above claims, wherein step (c) comprises a step of determining a difference in concentration of the gas component between the first and second positions.

5. The method according to any one of the above claims, wherein:
steps (a) and (b) each comprise a step of integrating the determined concentration of the gas component over a plurality of measuring cycles, and
step (c) comprises a step of determining the differential concentration of the gas component from the integrated first and second concentrations; and/or
step (c) comprises a step of integrating the differential concentrations of the gas component over a plurality of measuring cycles.

6. The method according to any one of the above claims, further comprising a step of
(e) providing an alarm and/or initiating a predefined action, if the determined differential concentration of the gas component exceeds a threshold value.

7. The method of claim 6, wherein the threshold value is dependent on whether the concentration of the gas component is increased or decreased between the first and second positions within the gas flow.

8. The method of claim 6 or 7, wherein the threshold value is dependent on an absolute concentration of the gas component in the gas flow.

## Patentansprüche

1. Verfahren zum Kontrollieren eines Gasstroms (20) zu einem und von einem Patienten, welches die folgenden Schritte umfasst:
(a) Bestimmen einer ersten Konzentration (200) einer Gaskomponente an einer ersten Position innerhalb des Gasstroms,
(b) Bestimmen einer zweiten Konzentration (210) der Gaskomponente an einer zweiten Position innerhalb des Gasstroms, wobei
sich ein Patient zwischen der ersten und der zweiten Position befindet,
die erste Konzentration eine Konzentration der Gaskomponente im eingeatmeten Gas repräsentiert,
die zweite Konzentration eine Konzentration der Gaskomponente im ausgeatmeten Gas repräsentiert, und
(c) Bestimmen (220) einer differentiellen Konzentration (230) der Gaskomponente aus der ersten und zweiten Konzentration, wobei die differentielle Konzentration anzeigt, ob die Konzentration der Gaskomponente zwischen der ersten und der zweiten Position innerhalb des Gasstroms zunimmt, abnimmt oder im Wesentlichen unverändert bleibt.

2. Verfahren nach Anspruch 1, bei dem sich die erste und die zweite Position an derselben Stelle, doch mit verschiedenen Richtungen des Gasstroms, oder an verschiedenen Stellen, doch mit derselben Richtung des Gasstroms, befinden.

3. Verfahren nach Anspruch 1 oder 2, bei dem:
die erste Konzentration eine Eingangskonzentration vor dem Passieren eines Objekts ist, das sich innerhalb des Gasstroms zwischen der ersten und der zweiten Position befindet, die zweite Konzentration eine Ausgangskonzentration nach dem Passieren des Objekts ist und das Verfahren des Weiteren den folgenden Schritt umfasst:
(d) Bestimmen der Art des Objekts mittels der differentiellen Konzentration der Gaskomponente, vorzugsweise so, dass:
das Objekt eine Quelle der Gaskomponente darstellt, falls die Konzentration der Gaskomponente ansteigt, oder
das Objekt die Gaskomponente zumindest teilweise dem Gasstrom entzieht, falls sich die Konzentration der Gaskomponente verringert.

4. Verfahren nach einem der vorhergehenden Ansprüche, bei dem der Schritt (c) einen Schritt umfasst, in dem eine Differenz der Konzentration der Gaskomponente zwischen der ersten und der zweiten Position ermittelt wird.

5. Verfahren nach einem der vorhergehenden Ansprüche, bei dem:
die Schritte (a) und (b) jeweils einen Schritt umfassen, in dem die ermittelte Konzentration der Gaskomponente über eine Vielzahl von Messzyklen integriert wird, und der Schritt (c) einen Schritt umfasst, in dem die differentielle Konzentration der Gaskomponente aus der integrierten ersten und der integrierten zweiten Konzentration bestimmt wird, und/oder der Schritt (c) einen Schritt umfasst, in dem die differentiellen Konzentrationen der Gaskomponente über eine Vielzahl von Messzyklen integriert werden.

6. Verfahren nach einem der vorhergehenden Ansprüche, welches des Weiteren den folgenden Schritt umfasst:
(e) Ausgeben eines Warnsignals und/oder Auslösen einer vorgegebenen Maßnahme, wenn die ermittelte differentielle Konzentration der Gaskomponente einen Schwellenwert überschreitet.

7. Verfahren nach Anspruch 6, bei dem der Schwellenwert davon abhängt, ob die Konzentration der Gaskomponente zwischen der ersten und der zweiten Position innerhalb des Gasstroms ansteigt oder sich verringert.

8. Verfahren nach Anspruch 6 oder 7, bei dem der Schwellenwert von einer absoluten Konzentration der Gaskomponente im Gasstrom abhängt.

## Revendications

1. Procédé de monitoring d'un flux gazeux (20) passant vers et depuis un patient, procédé comprenant les étapes suivantes :
(a) détermination d'une première concentration (200) d'un composant gazeux dans une première position dans le flux gazeux;
(b) détermination d'une deuxième concentration (210) du composant gazeux dans une deuxième position dans le flux gazeux,
dans lequel le patient est situé entre lesdites première et deuxième positions,
la première concentration représentant la concentration en gaz d'inhalation dudit composant gazeux,
la deuxième concentration représentant une concentration en gaz d'exhalation dudit composant gazeux; et
(c) détermination (220) d'une concentration différentielle (230) du composant gazeux à partir des première et deuxième concentrations, la concentration différentielle indiquant si la concentration du composant gazeux est augmentée, réduite ou est restée essentiellement inchangée entre les première et deuxième positions dans le flux gazeux.

2. Procédé selon la revendication 1, dans lequel les première et deuxième positions sont prévues
au même endroit mais avec des directions différentes du flux gazeux ou
à des endroits différents mais dans la même direction du flux gazeux..

3. Procédé selon l'une des revendications 1 ou 2, dans lequel
la première concentration est une concentration d'admission avant de passer un objet situé dans le flux gazeux entre les première et deuxième positions ;
la deuxième concentration est une concentration de sortie après avoir passé l'objet et
le procédé comprend par ailleurs les étapes suivantes :
(d) détermination de la caractéristique de l'objet au moyen de la concentration différentielle du composant gazeux, de préférence de telle manière que :
l'objet représente une source de composant gazeux au cas où la concentration du composant gazeux a augmenté ou
l'objet élimine, du moins partiellement, le composant gazeux du flux gazeux au cas où la concentration du composant gazeuse est réduite.

4. Procédé selon l'une quelconque des revendications précédentes dans lequel l'étape (c) comprend une étape de détermination d'une différence de concentration du composant gazeux entre les première et deuxième positions.

5. Procédé selon l'une quelconque des revendications précédentes, dans lequel les étapes (a) et (b) comprennent chacune une étape d'intégration de la concentration déterminée du composant gazeux sur plusieurs cycles de mesure et l'étape (c) comprend une étape de détermination de la concentration différentielle du composant gazeux à partir des premières et deuxième concentrations intégrées; et/ou
l'étape (c) comprend une étape d'intégration des concentrations différentielles du composant gazeux sur une pluralité de cycles de mesure.

6. Procédé selon l'une des revendications précédentes, comprenant par ailleurs une étape visant à
(e) fournir une alarme et/ou à déclencher une action préalablement déterminée si la concentration différentielle déterminée du composant gazeux dépasse une valeur seuil.

7. Procédé selon la revendication 6 dans lequel la valeur-seuil dépend du fait que la concentration du composant gazeux soit augmentée ou réduite entre les première et deuxième positions dans le flux gazeux.

8. Procédé selon l'une des revendications 6 ou 7, dans lequel la valeur-seuil dépend d'une concentration absolue du composant gazeux dans le flux gazeux.
